# EUROPEAN PATENT SPECIFICATION

(11) **EP 2 262 898 B1**
(45) Date of publication and mention of the grant of the patent: **24.01.2018**
(21) Application number: 09718915.3
(22) Date of filing: 06.03.2009
(51) Int. Cl.: A01K 67/027, C12N 15/85, C12N 15/90, C12N 5/10, A61D 19/00, C12N 5/16

(54) **ES CELL-DERIVED MICE FROM DIPLOID HOST EMBRYO INJECTION**
VON ES-ZELLEN ABSTAMMENDE MÄUSE AUS DER INJEKTION EINES DIPLOIDEN WIRTSEMBRYOS
SOURIS ISSUES DE CELLULES SOUCHES EMBRYONNAIRES PAR INJECTION D'EMBRYONS HÔTES DIPLOÏDES

(30) Priority: 07.03.2008 US 34807 P
(43) Date of publication of application: 22.12.2010
(73) Proprietor: Regeneron Pharmaceuticals, Inc., Tarrytown, NY 10591 (US)
(72) Inventor: POUEYMIROU, William, White Plains, NY 10607 (US); DECHIARA, Thomas, M., Rye, NY 10580 (US); AUERBACH, Wojtek, Ridgewood, NJ 07450 (US); ECONOMIDES, Aris, N., Tarrytown, NY 10591 (US); GALE, Nicholas, W., Yorktown Heights, NY 10598 (US); FRENDEWEY, David, New York, NY 10016 (US); VALENZUELA,David, M., Yorktown Heights, NY 10598 (US)
(74) Representative: J A Kemp
(86) International application number: PCT/US2009/036276
(87) International publication number: WO 2009/114400

(56) References cited:
- WO-A-2006/044962
- WO-A-2008/112542
- WO-A1-2006/025802
- POUEYMIROU WILLIAM T ET AL: "F0 generation mice fully derived from gene-targeted embryonic stem cells allowing immediate phenotypic analyses" NATURE BIOTECHNOLOGY, NATURE PUBLISHING GROUP, NEW YORK, NY, US, vol. 25, no. 1, 24 December 2006 (2006-12-24), - January 2007 (2007-01) pages 91-99, XP002464122 ISSN: 1087-0156
- SATO M, TANIGAWA M.: "Production of CETD transgenic mouse line allowing ablation of any type of specific cell population." MOL REPROD DEV., vol. 72, no. 1, September 2005 (2005-09), pages 54-67, XP002531697
- MATSUMURA H, HASUWA H, INOUE N, IKAWA M, OKABE M.: "Lineage-specific cell disruption in living mice by Cre-mediated expression of diphtheria toxin A chain" BIOCHEM BIOPHYS RES COMMUN, vol. 321, no. 2, 20 August 2004 (2004-08-20), pages 275-279, XP002531698
- BROCKSCHNIEDER D, LAPPE-SIEFKE C, GOEBBELS S, BOESL MR, NAVE KA, RIETHMACHER D.: "Cell depletion due to diphtheria toxin fragment A after Cre-mediated recombination." MOL CELL BIOL., vol. 24, no. 17, September 2004 (2004-09), pages 7636-7642, XP002531699
- MITSUI K ET AL: "The homeoprotein nanog is required for maintenance of pluripotency in mouse epiblast and ES cells" CELL, CELL PRESS, CAMBRIDGE, NA, US, vol. 113, no. 5, 1 May 2003 (2003-05-01), pages 613-642, XP002904478 ISSN: 0092-8674
- HONG Y, WINKLER C, LIU T, CHAI G, SCHARTL M.: "Activation of the mouse Oct4 promoter in medaka embryonic stem cells and its use for ablation of spontaneous differentiation" MECH DEV., vol. 121, no. 7-8, July 2004 (2004-07), pages 933-943, XP002531701
- AMAR M. SINGH, TAKASHI HAMAZAKI, KATHERINE E. HANKOWSKI, NAOHIRO TERADA, M.D.,: "A Heterogeneous Expression Pattern for Nanog in Embryonic Stem Cells" STEM CELLS, vol. 25, 5 July 2007 (2007-07-05), pages 2534-2542, XP002531703
- HAYASHI S1, TENZEN T, MCMAHON AP.: "Maternal inheritance of Cre activity in a Sox2Cre deleter strain.", GENESIS. 2003 OCT, vol. 37, no. 2, October 2003 (2003-10), pages 51-53,
- HAYASHI S ET AL: "Efficient gene modulation in mouse epiblast using a Sox2Cre transgenic mouse strain", MECHANISMS OF DEVELOPMENT, ELSEVIER SCIENCE IRELAND LTD, IE, vol. 119, 1 December 2002 (2002-12-01), pages S97-S101, XP027424381, ISSN: 0925-4773, DOI: 10.1016/S0925-4773(03)00099-6 [retrieved on 2002-12-01]
- GRIDLEY T ET AL: "Laser surgery for mouse geneticists", NAT BIOTECHNOL,, vol. 25, no. 1, 24 December 2006 (2006-12-24), pages 59-60, XP002531700, DOI: 10.1038/NBT0107-59
- Da Yong Wu ET AL: "Isolation and characterization of the murine Nanog gene promoter", CELL RESEARCH - XIBAO YANJIU, vol. 15, no. 5, 1 May 2005 (2005-05-01), pages 317-324, XP055244582, GB, CN ISSN: 1001-0602, DOI: 10.1038/sj.cr.7290300
- BROCKSCHNIEDIER DAMIAN ET AL: "An improved mouse line for cre-induced cell ablation due to diphtheria toxin A, expressed from the Rosa26 locus", GENESIS: THE JOURNAL OF GENETICS AND DEVELOPMENT, WILEY-LISS, NEW YORK, NY, US, vol. 44, no. 7, 1 July 2006 (2006-07-01), pages 322-327, XP009130404, ISSN: 1526-954X, DOI: 10.1002/DVG.20218 [retrieved on 2006-06-21]
- OKITA K ET AL: "Generation of germline-competent induced pluripotent stem cells", NATURE, NATURE PUBLISHING GROUP, UNITED KINGDOM, vol. 448, no. 7151, 19 July 2007 (2007-07-19), pages 313-317, XP002555950, ISSN: 0028-0836, DOI: 10.1038/NATURE05934 [retrieved on 2007-06-06]

## Description

### FIELD

Making genetically modified non-human animals by altering their genomes; making non-human animals having a genetic contribution from a non-human donor cell by combining the non-human donor cell with a non-human host embryo.

### BACKGROUND

Methods for modifying eukaryotic cells are known in the art. See, for example, U.S. Patent No. 6,586,251. Methods for generating animals having contributions from donor cells by combining donor cells and host embryos are also known in the art. See, for example U.S. Patent No. 7,294,754. There is a need in the art for further methods for generating animals having contributions from donor cells by exposing a host embryo to donor cells.

### BRIEF SUMMARY

The invention provides a mouse embryo, comprising a cell having in its genome:
(a) a site-specific recombinase gene operably linked to a Nanog promoter that expresses the site-specific recombinase gene in a cell of the inner cell mass (ICM) but not the trophectoderm;
(b) a gene encoding a toxin, operably linked to a promoter, wherein the toxin gene is present at an expression-permissive locus; and
(c) a nucleic acid sequence that prevents expression of the gene encoding the toxin, wherein the nucleic acid sequence is located between the promoter for the gene encoding the toxin and the coding sequence of the gene encoding the toxin and is flanked on both sides by recombinase recognition sites such that in the presence of the site-specific recombinase the nucleotide sequence is removed and the promoter for the gene encoding the toxin drives transcription of the gene encoding the toxin.

The invention further provides a method for making a mouse or mouse embryo from a mouse donor cell and a host embryo, comprising:
(a) introducing mouse donor cells into a mouse host embryo, wherein the mouse donor cells are selected from the group consisting of embryonic stem (ES) cells, pluripotent stem (PS) cells, or induced pluripotent stem (iPS) cells, and wherein the host embryo is a pre-morula stage embryo and comprises
   (i) a site-specific recombinase gene operably linked to a Nanog promoter that expresses the site-specific recombinase gene in a cell of the inner cell mass (ICM) but not the trophectoderm; and
   (ii) a gene encoding a toxin, operably linked to a promoter, wherein the toxin gene is present at an expression-permissive locus; and
   (iii) a nucleic acid sequence that prevents expression of the gene encoding the toxin, wherein the nucleic acid sequence is located between the promoter for the gene encoding the toxin and the coding sequence of the gene encoding the toxin and is flanked on both sides by recombinase recognition sites such that in the presence of the site-specific recombinase the nucleotide sequence is removed and the promoter for the gene encoding the toxin drives transcription of the gene encoding the toxin;
(b) gestating the embryo of step (a) in a pseudopregnant mouse.

The invention additionally provides a breeding pair of genetically modified mice that, when mated, generate an embryo comprising a first nucleic acid construct and a second nucleic acid construct in its genome, said breeding pair comprising a first mouse comprising a first nucleic acid construct in its genome and a second mouse comprising a second nucleic acid construct in its genome,
wherein the first nucleic acid construct comprises a site-specific recombinase gene operably linked to a Nanog promoter that expresses the site-specific recombinase gene in a cell of the inner cell mass (ICM) but not the trophectoderm, and
wherein the second nucleic acid construct comprises a gene encoding a toxin and a nucleic acid sequence that prevents expression of the gene encoding the toxin, wherein the gene encoding the toxin is operably linked to a promoter and present at an expression-permissive locus, wherein the nucleic acid sequence is located between the promoter for the gene encoding the toxin and the coding sequence of the gene encoding the toxin and is flanked on both sides by recombinase recognition sites such that in the presence of the site-specific recombinase the nucleotide sequence is removed and the promoter for the gene encoding the toxin drives transcription of the gene encoding the toxin.

The invention also provides a mouse comprising the mouse embryo of the invention.

Methods and compositions are described herein for making a non-human animal or embryo by introducing a non-human donor cell into a non-human host embryo and growing the embryo in a non-human host animal to obtain an animal that is donor-cell-derived, including non-human embryos or animals that are donor-cell-derived in whole or in substantial part.

Compositions and methods for killing, inactivating, blocking differentiation of, or rendering ICM cells of a non-human embryo incapable of proliferating are also described herein, as well as DNA constructs and genetically modified animals for making non-human host embryos that are capable of generating an ICM whose cells cannot proliferate or differentiate or contribute to a developing embryo.

Compositions and methods are also described herein for making non-human embryos wherein the ICM is absent or incapable of proliferating or substantially incapable of proliferating, and wherein the non-human embryos are suitable for receiving non-human donor cells that are capable of populating the embryo.

In a first aspect of the disclosure, a mouse embryo is described herein, comprising a cell having in its genome (a) a site-specific recombinase gene operably linked to a developmentally-regulated promoter that expresses the site-specific recombinase gene in a cell of the inner cell mass (ICM) during an embryo stage; and, (b) a gene whose expression prevents proliferation of an ICM cell, wherein expression of the gene whose expression prevents proliferation of the ICM cell is induced by the presence of the site-specific recombinase.

The site-specific recombinase may be selected from the group consisting of Cre, a modified Cre, Dre, Flp, Flpe, Flp(o), and phiC31. In a specific embodiment, the site-specific recombinase is Cre.

In one aspect of the disclosure, the developmentally-regulated promoter is a promoter for a gene that is not transcribed in nature in a wild-type animal prior to an embryo stage selected from a four-cell stage, an 8-cell stage, a 16-cell stage, a 32-cell stage, a 64-cell stage, a blastocyst stage wherein the primitive endoderm is not substantially formed, and a blastocyst stage wherein the primitive endoderm is unformed. In one aspect of the disclosure, the developmentally-regulated promoter is a promoter for a gene that is not transcribed in nature in a wild-type animal prior to an embryo stage selected from a Theiler Stage (TS) TS2, TS3, TS4, TS5, and TS6.

In one aspect of the disclosure, the developmentally-regulated promoter is selected from a promoter for one of the following genes, or a transcriptionally-effective fragment thereof: Nanog, Oct3/4, Sox2, Klf4, Fgf4, Rex1, Cripto, Dax, Esg1, Nat1, and Fbx15. In a specific embodiment, the promoter is a Nanog promoter. A transcriptionally-effective fragment includes fragments of promoters of the above-mentioned genes which have not been rendered incapable of supporting transcription of a gene operably linked to the fragment.

The mouse embryo can be selected from an embryo of an inbred strain, a hybrid strain, an outbred strain, and a mixed strain. Specifically, the strain of mouse is selected from a 129 strain, a BALB/c strain, a C57BL/6 strain, and a hybrid of any two of the aforementioned strains. The strain of mouse can be selected from a mix of any of the aforementioned strains. In a specific aspect of the disclosure, the mouse is a mix of the C57BL/6 and 129 strains. In a specific aspect of the disclosure, the mouse is an outbred strain selected from Swiss Webster, ICR, CD1, and MF1.

The host embryo exhibits any ploidy. In one aspect of the disclosure, the host embryo is selected from a diploid host embryo and a tetraploid host embryo. In a specific aspect of the disclosure, the host embryo is a diploid host embryo.

In one aspect of the disclosure, the embryo stage is selected from a 1-cell stage, a 2-cell stage, a 4-cell stage, an 8-cell stage, a 16-cell stage, a 32-cell stage, and a 64-cell stage. In one aspect of the disclosure, the embryo is in a stage selected from a pre-morula stage, a morula stage, an uncompacted morula stage, and a compacted morula stage. In one aspect of the disclosure, the embryo stage is selected from a Theiler Stage 1 (TS1), a TS2, a TS3, a TS4, a TS5, and a TS6, with reference to the Theiler stages described in Theiler (1989) "The House Mouse: Atlas of Mouse Development," Springer-Verlag, New York. In a specific aspect of the disclosure, the Theiler Stage is selected from TS1, TS2, TS3, and a TS4. In one aspect of the disclosure, the embryo stage is a blastocyst stage. In a specific aspect of the disclosure, the embryo stage is a blastocyst stage, wherein primitive endoderm is as yet unformed or is as yet substantially unformed. In one aspect of the disclosure, the formation of the primitive endoderm is no more than 1% complete, in another aspect no more than 5% complete, in another aspect no more than 10% complete, in another aspect no more than 25% complete, in another aspect no more than 50% complete, in another aspect no more than 75% complete.

In one aspect of the disclosure, the gene whose expression prevents proliferation of an ICM cell is selected from a microRNA, an siRNA, a gene encoding a toxin or toxically effective fragment thereof, diphtheria toxin A fragment (DTA), attenuated DTA, tox-176, exotoxin-A, PE 40, herpes simplex virus 1 thymidine kinase, ricin, Shiga toxin, and a gene encoding a receptor for a toxin or a toxin-binding fragment thereof. In a specific embodiment, the toxin is DTA. In one aspect of the disclosure, the gene whose expression prevents proliferation of an ICM cell is a fusion protein comprising a toxin domain and a ligand-binding domain wherein in the presence of a ligand binding to the ligand-binding domain (but not in the absence of ligand), the toxic domain is toxic to the ICM cell. In one aspect of the disclosure, the fusion protein comprises a toxin domain and a ligand-binding domain wherein in the absence of a ligand binding to the ligand-binding domain (but not in the presence of ligand), the toxic domain is toxic to the ICM cell. In a specific aspect of the disclosure, the fusion protein is selected from DTA-ERT2 and caspase3-ERT2 and the ligand is tamoxifen.

The site-specific recombinase induces expression of the gene whose expression prevents proliferation of the ICM by removing a nucleic acid sequence between a promoter operably linked to the gene whose expression prevents proliferation of the ICM and the gene whose expression prevents proliferation of the ICM. In a specific aspect, the gene whose expression prevents proliferation of the ICM is a DTA gene, the promoter is a Rosa promoter, and the nucleotide sequence between the promoter and the DTA gene is flanked on both sides by IoxP sites, such that in the presence of Cre the loxed nucleotide sequence is removed and the Rosa promoter is then capable of driving transcription of the DTA gene.

In the aspects described below, the site-specific recombinase, the developmentally-regulated promoter, the embryo, the ploidy of the embryo, the embryo stage, the gene whose expression prevents proliferation of an ICM cell, the toxin, the gene encoding the toxin or toxically effective fragment thereof, the fusion protein, and the promoter that is capable of driving transcription of the gene encoding the toxin, include the aspects described above for the first aspect of the disclosure, unless otherwise specified or unless excluded by the context of the recited aspects that follow.

A method for making a mouse or mouse embryo from a mouse donor cell and a host embryo is described herein, comprising: (a) introducing a mouse donor cell into a mouse host embryo, wherein the host embryo comprises (i) a site-specific recombinase gene operably linked to a developmentally-regulated promoter that expresses the site-specific recombinase gene in a cell of the inner cell mass (ICM) during an embryo stage; and, (ii) a gene whose expression prevents proliferation of an ICM cell, wherein the expression of the gene whose expression prevents proliferation of the ICM cell is induced by the presence of the site-specific recombinase; (b) introducing the mouse donor cell into the host embryo of step (a); and, (c) gestating the embryo of step (b) in pseudopregnant mouse.

The mouse donor cell is selected from an ES cell, a PS cell, and an iPS cell. In the aspects and embodiments described below, donor cells include ES cells, PS cells, and iPS cells, unless otherwise specified or unless ES cells, PS cells, and iPS cells are excluded by the context of the recited aspects or embodiments that follow.

In one aspect, a non-human embryo is provided that lacks an ICM or comprises an ICM that is incapable of proliferating or substantially incapable of proliferating.

In one aspect, the embryo is capable of serving as a host embryo for receiving non-human donor cells that are capable of populating the embryo. In a specific aspect of the disclosure, the cells of the host embryo are diploid. In a specific aspect of the disclosure, the embryo is diploid, comprises a trophectoderm, and is at a pre-gastrulation stage.

In one aspect of the disclosure, the embryo is in a cryotube. In another aspect of the disclosure, the embryo is in a medium comprising a cryoprotectant. In one aspect of the disclosure, the embryo is maintained in a medium comprising a cryoprotectant, wherein the container is in contact with liquid nitrogen.

In one aspect, a method for making a non-human embryo suitable as a host embryo for non-human donor cells is described herein, the method comprising physically removing the ICM. In a specific embodiment, physically removing the ICM comprises extracting all or substantially all of the ICM using a syringe or a needle. In a specific aspect, the embryo is at a pre-gastrulation stage and comprises a trophectorderm and an ICM.

In one aspect, a method for making a non-human embryo suitable as a host embryo for non-human donor cells is described herein, the method comprising chemically killing ICM cells or chemically rendering them incapable of proliferating. In one aspect, the embryo is at a pre-gastrulation stage and comprises a trophectoderm and an ICM. In one aspect, chemically killing ICM cells comprises a method wherein the embryo is exposed to a substance that kills ICM cells but does not substantially harm trophectoderm cells. In one aspect, chemically killing ICM cells comprises exposing them to an agent that forms a covalent bond with a component of an ICM or breaks a covalent bond in a component of an ICM, wherein forming or breaking a covalent bond in a component of the ICM renders the ICM incapable of proliferating. In a specific aspect, the agent is injected into the ICM. In another specific aspect, the trophectoderm is made permeable to the agent and the embryo is soaked in a medium comprising the agent. In one aspect, the agent is a non-protein and non-nucleic acid substance.

In one aspect, a method for making a non-human embryo suitable as a host embryo for non-human donor cells is described herein, wherein the embryo comprises an ICM, comprising: exposing the embryo to a physical condition that is preferentially deleterious to cells of the ICM but not the trophectoderm; or, exposing the ICM to an antibody conjugated to an agent that is toxic to ICM cells, wherein the antibody recognizes an antigen on ICM cells.

In one aspect the toxin or antibody is substantially incapable of binding to an antigen on cells of the trophectoderm. In one aspect, the agent that is toxic to the cells of the ICM is nontoxic to cells of the trophectoderm, or is insufficiently toxic to the cells of the trophectoderm such that the trophectoderm remains functional whereas the ICM cells are ablated or rendered incapable of proliferating. In another aspect, the method comprises making a genetically modified mouse that comprises a receptor for a toxin or antibody, wherein the receptor is expressed by ICM cells, and exposing an embryo of the genetically modified mouse to the toxin or antibody, wherein the ICM cells are killed or rendered incapable of proliferating.

In one aspect, a genetically modified mouse or embryo is described herein, wherein the genetically modified mouse or embryo comprises a gene encoding an activator, wherein the activator is expressed during an embryo stage. The activator of this aspect, and of the other aspects described herein, can include, according to the context, a site-specific recombinase as described in other aspects and embodiments herein. In various aspects the activator of this and other aspects can include, according to the context, a protein that is expressed in the ICM but is not expressed in the trophectoderm.

In one aspect, the gene encoding the activator is operably linked to a developmentally-regulated promoter that expresses the gene encoding the activator during an embryo stage.

In one aspect, the activator comprises a protein. In one aspect, the protein is a site-specific recombinase as described herein.

In one aspect, the developmentally-regulated promoter is a promoter that drives expression in ICM cells but not in trophectoderm cells. In a specific aspect, the activator is a Cre recombinase and the developmentally-regulated promoter is a Nanog gene promoter.

In one aspect, the genetically modified mouse is homozygous for a Cre recombinase gene operably linked to a developmentally-regulated promoter.

In one aspect, the embryo stage is selected from a stage in which the promoter for one of the following genes is active: Nanog, Oct3/4, Sox2, Klf4, Fgf4, Rex1, Cripto, Dax, Esg1, Nat1 and Fbx15.

In one aspect, a genetically modified embryo or mouse is described herein, wherein the genetically modified embryo or mouse comprises a gene whose expression is toxic to a cell (i.e., a toxic gene), wherein the gene toxic to a cell is present at an expression-permissive locus and wherein the gene toxic to the cell is incapable of being expressed in the absence of an activator.

In various aspects, the gene whose expression is toxic to a cell includes a gene whose expression prevents proliferation of an ICM cell.

In one aspects, the gene toxic to a cell is conditionally incapable of expression due to a nucleic acid sequence that prevents expression of the toxic gene. In one aspect the nucleic acid sequence that prevents expression of the toxic gene is located between a promoter and the coding sequence of the toxic gene. In a specific aspect, the nucleic acid sequence that prevents expression of the toxic gene is flanked on both sides by a recombinase recognition site, such that in the presence of a recombinase that recognizes the recombinase recognition sites, the loxed nucleotide sequence is removed and the promoter is then capable of driving transcription of the toxic gene. In a specific aspect, the promoter is a Rosa promoter.

In one aspect, the sequence that prevents expression of the toxic gene comprises a transcription termination sequence located between the promoter and the coding sequence of the toxic gene and flanked on both sides by a recombinase recognition site.

In one aspect, the sequence flanked on both sides by the recombinase recognition site is a sequence for a nucleic acid or protein followed by a transcription termination sequence. In a specific embodiment, the sequence codes for a fluorescent protein such as, for example, green fluorescent protein (GFP), eGFP, CFP, YFP, eYFP, BFP, eBFP, DsRed, and MmGFP; or is selected from a group of genes that impart resistance to an antibiotic/drug, such as neomycin phosphotransferase (neo^{r}), hygromycin B phosphotransferase (hyg^{r}), xanthine/guanine phosphoribosyl transferase (gtp), or fusions thereof.

In a specific aspect, the toxic gene is a sequence encoding a protein that is toxic to a cell, such as DTA, and between the promoter and the toxic protein coding sequence (e.g., between the promoter and the DTA coding sequence) is a IoxP-marker gene-transcription termination signal IoxP (floxed marker-stop), and preceding the floxed marker-termination signal is a sequence encoding a 3' splice site (splice acceptor) that facilitates operable linkage of the DTA to a promoter upon removal of the floxed marker-termination sequence by Cre recombinase.

In one aspect, the promoter is a promoter that is inserted to be in proximity to the DTA coding sequence and is capable of driving expression of DTA in the presence of activator. In another aspect, the promoter is at a locus where the promoter is typically found in nature.

In one aspect, the toxic gene is placed at an expression-permissive locus selected by random insertion of a nucleic acid construct comprising a marker gene and the toxic gene, by introducing the nucleic acid construct into a cell (e.g, an ES or PS cell employed as a donor cell to make the modified mouse or embryo) and screening cells for expression of the marker.

In one aspect, the expression-permissive locus is selected by introducing a nucleic acid construct comprising a toxic gene and homology arms directing the toxic gene to a pre-selected or specific locus. In a specific aspect the expression-permissive locus is the Gt(ROSA)26Sor locus.

In one aspect, a breeding pair of genetically modified mice is described herein that, when mated, generate an embryo that comprises (a) an activator locus, comprising a sequence encoding an activator, operably linked to a developmentally-regulated promoter; and (b) a responder locus, comprising a gene that is toxic to a cell and a sequence that prevents expression of the gene that is toxic to the cell; wherein the activator is capable of modifying the responder locus to cause expression of the gene that is toxic to the cell.

In one aspect, the activator locus comprises a sequence that encodes an activator protein. In one aspect, the responder locus comprises a sequence that encodes a responder. The responder of this aspect, and of the other aspects described herein employing a responder, can include, according to the context, a toxic nucleic acid sequence (e.g., encoding a toxic microRNA or a toxic protein) described in other aspects herein. In one aspect, the responder locus encodes a toxin, e.g., DTA. In one aspect, the responder locus comprises a sequence that prevents transcription of the toxic nucleic acid sequence in the absence of the activator, but in the presence of the activator the sequence no longer prevents transcription of the toxic nucleic acid. In a specific aspect, the responder locus comprises a promoter and the sequence of the toxic nucleic acid, having a transcription inhibiting sequence flanked on both sides by recombinase recognition sites positioned between the toxic nucleic acid and the promoter.

In one aspect, the mice are each independently selected from the strains described herein.

In one aspect, a mouse embryo is described herein that comprises (a) an activator locus, comprising a gene encoding an activator, wherein the gene encoding the activator is operably linked to a developmentally-regulated promoter; and (b) a responder locus, comprising a gene that is toxic to a cell and a sequence that prevents expression of the gene that is toxic to the cell, wherein the activator is capable of modifying the responder locus to cause expression of the gene that is toxic to the cell.

In one aspect, the mouse embryo is at a developmental stage wherein the developmentally-regulated promoter is expressing the activator gene.

In one aspect, the mouse embryo is a blastocyst that comprises ICM cells that are incapable of proliferating or substantially incapable of proliferating. In another aspect, the blastocyst comprises a donor mouse cell that has been introduced into the blastocyst. In a specific aspect, the donor cell is selected from a mouse ES cell and a pluripotent stem (PS) cell, for example an induced pluripotent stem (iPS) cell. In one aspect, the donor mouse cell is genetically modified.

In one aspect, a method is described herein for making a mouse or mouse embryo, from a mouse donor cell and a host embryo, comprising: (a) introducing a mouse donor cell into a mouse host embryo, wherein the host embryo comprises (i) an activator locus comprising a gene encoding an activator, wherein the activator is capable of activating expression at a responder locus; and (ii) a responder locus, comprising a toxic gene that is expressed only in the presence the activator; (b) introducing a donor mouse cell into the host embryo of step (a); and, (c) gestating the embryo of step (b) in a pseudopregnant mouse.

In one aspect, the activator locus comprises a sequence that encodes an activator protein. In one aspect, the responder locus comprises a sequence that encodes a responder protein.

In one aspect the toxic gene is expressed only in the presence of the activator, and the activator gene is expressed at a stage during which the Nanog gene is expressed, and wherein the activator gene is not expressed in the trophectoderm.

In one aspect, a method is described herein for making a mouse, comprising (a) providing a host mouse embryo, wherein the host mouse embryo comprises an ICM, and wherein the cells of the ICM comprise a toxic gene and an activator gene, wherein expression of the activator gene is controlled by a developmentally-regulated promoter that is active during the blastocyst stage in cells of the ICM but not the trophectoderm; (b) introducing a donor ES cell into the host embryo; and (c) introducing the host embryo comprising the donor ES cell into a mouse under conditions suitable for gestating the host embryo comprising the donor ES cell; and (d) allowing the host embryo comprising the donor ES cell to develop into a mouse.

In one aspect, a method is described herein for making a mouse, comprising (a) introducing into a diploid mouse host blastocyst a mouse donor ES or PS cell, wherein the host blastocyst comprises an ICM, and wherein the cells of the host ICM are incapable of proliferating; and (b) allowing the blastocyst of (a) comprising the mouse donor ES or PS cell to develop into a mouse in a pseudopregnant female mouse.

In one aspect, the methods and compositions described herein are employed to make a mouse or a mouse embryo by introducing one or more donor mouse cells into a mouse host embryo, wherein all tissues of the mouse or mouse embryo that is made are no less than 90% derived from the donor cells, no less than 95% derived from the donor cells, no less than 98% derived from the donor cells, no less than 99% derived from the donor cells, or 100% derived from the donor cells.

In one aspect, the methods and compositions described herein are employed to make a mouse or a mouse embryo by introducing one or more donor cells into a mouse host embryo, and gestating the embryo to form a resulting mouse, wherein the resulting mouse is no more than 3% derived from the host embryo, no more than 2% derived from the host embryo, no more than 1% derived from the host embryo, no more than 0.5% derived from the host embryo, no more than 0.2% derived from the host embryo, no more than 0.1% derived from the host embryo, no more than 0.05% derived from the host embryo.

In one aspect, methods and compositions are described herein for making a mouse progeny derived from a donor cell, comprising introducing one, two, three, four, five, six, seven, eight, nine, ten, eleven, twelve, thirteen, fourteen, fifteen or sixteen, cells into a host embryo that is a fertilized egg or is at a 2-cell, 4-cell, 8-cell, 16-cell, 32-cell stage, or a blastocyst; introducing the embryo into a mouse that is capable of gestating the embryo, wherein the mouse progeny is no more than 0.2% or 0.1% derived from the host embryo. In one aspect, the host embryo is a blastocyst and the number of donor cells is 12-16 cells. In one aspect, the host embryo is at the 8-cell stage or an earlier stage and the number of donor cells is 1-10, in a specific aspect 2-10 cells.

In one aspect, a method is described herein employing any combination of the compositions and/or methods described herein to make a mouse from a donor ES or PS cell and a host embryo, wherein the mouse is in the F0 generation and is 99.8%, 99.9%, or 100% derived from the donor ES or PS cell. In a specific aspect, the donor cell is an ES or PS cell, the embryo is diploid and at a blastocyst stage wherein the blastocyst comprises an ICM, and wherein the cells of the ICM are ablated or are incapable of proliferating due to expression of a toxic gene, and wherein the donor cell is introduced into the diploid embryo at a stage selected from a 2-cell stage, a 4-cell stage, an 8-cell stage, a 16-cell stage, a 32-cell stage, and a blastocyst stage.

In one aspect, a mouse host embryo is described herein, wherein the mouse host embryo comprises an ICM and a trophectoderm, wherein the cells of the ICM are incapable of proliferating and wherein the cells of the ICM express a gene that is toxic to ICM cells, and wherein the cells of the trophectoderm do not express the gene. In one aspect, the gene that is toxic to ICM cells encodes a protein that is toxic to ICM cells. In another aspect, the gene that is toxic to ICM cells expresses a microRNA that is toxic to ICM cells. In one aspect, the mouse host embryo further comprises a mouse donor cell selected from an ES cell and a PS cell. In a specific aspect, the mouse host embryo is diploid.

In one aspect, a mouse host embryo is described herein, wherein the embryo lacks an ICM that is derived from the mouse host embryo.

In one aspect, a mouse host embryo is described herein, wherein the host embryo comprises an ICM that is not capable of proliferating, wherein the embryo is capable of receiving donor cells and developing into a mouse derived from the donor cells.

In one aspect, a mouse host embryo is described herein, wherein the host embryo comprises ICM cells derived from the host and ICM cells derived from a donor, wherein the ICM cells derived from the host are incapable of contributing to development of the embryo.

In one aspect, a mouse host embryo is described herein, wherein the host embryo comprises an ICM, wherein all viable cells of the ICM are derived from a donor mouse.

In one aspect, an embryo is described herein, wherein the embryo comprises a genetic modification that renders the ICM incapable of contributing to development of the embryo into a live born animal. In one aspect, the genetic modification that renders the ICM incapable of contributing to the development of the embryo is a modification of a gene whose transcription is essential for embryogenesis. In one aspect, the genetic modification comprises a heterozygous or homozygous mutation in a Ronin gene, a Nanog gene, an Oct3/4 gene, a Sox2 gene, a Klf4 gene, a Fgf4 gene, a Rex1 gene, a Cripto gene, a Dax gene, a Esg1 gene, a Nat1 gene, and a Fbx15 gene. In a specific aspect, the genetic modification comprises a Ronin gene knockout of a knockout of at least one of the aforementioned genes. In a specific aspect, the genetic modification is a conditional knockout.

In one aspect, a method for making a mouse derived in whole or substantial part from a donor cell, comprising introducing a donor cell into a mouse embryo, wherein the mouse embryo is substantially incapable of expressing a functional Ronin protein. In one aspect, the donor cell is selected from an ES, a PS, and an iPS cell. In one aspect, the donor cell comprises a genetic modification.

Any of the aspects and embodiments described herein can be combined with any other aspect or embodiment unless it is clear from the context that the aspect or embodiment is incompatible with another aspect or embodiment.

Other objects and advantages will become apparent from a review of the ensuing detailed description.

### BRIEF DESCRIPTION OF THE FIGURES

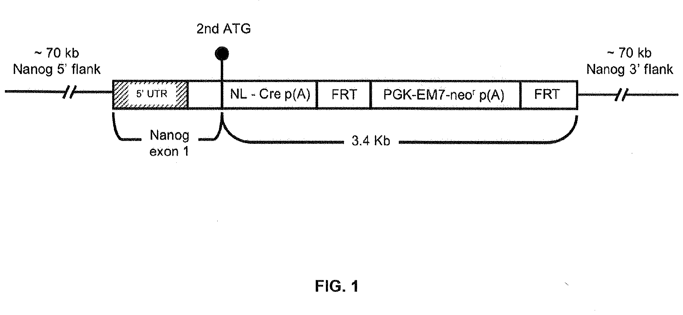

FIG. 1 is a schematic representation of a DNA construct (an activator construct) containing a recombinase gene useful in an embodiment of the invention. "5' UTR" refers to the part of the mouse Nanog gene that encodes the untranslated portion at the 5' end of the Nanog messenger RNA (mRNA). "Nanog exon 1" refers to the part of the Nanog gene that encodes the first exon of the Nanog precursor mRNA; "2nd ATG" refers to the part of the Nanog gene that encodes the second in-frame AUG codon in the Nanog mRNA. The Nanog protein coding sequence on mouse BAC clone 359m22 (Incyte Genomics BAC library 129/SvJ release 2) was deleted from the ATG to a position in the 3' UTR. "FRT" in each instance refers to a Flp recombinase recognition site. "PGK-EM7-neo^{r}" refers to a neomycin phosphotransferase coding sequence operably linked to the promoter of the mouse Pgk1 gene, for expression in mammalian cells, and to an EM7 promoter, for expression in bacterial cells. "NL-Cre" refers to the protein coding sequence of the Cre recombinase N-terminally tagged with a nuclear localization signal. The term "p(A)" or "polyA" refers to nucleic acid sequences that signal for transcription termination and mRNA polyadenylation. The notation "-70 kb Nanog 5' flank" and "3' flank" refer to sequences in the BAC clone that flank the Nanog protein coding sequence. Sequence lengths are not drawn to scale.

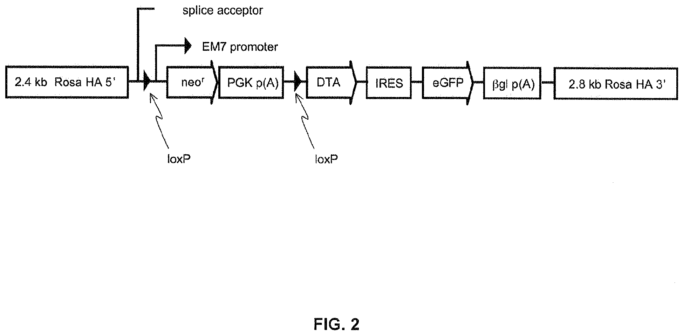

FIG. 2 is a schematic representation of a DNA construct (a responder construct) containing a toxic gene useful in an embodiment of the invention, where the toxic gene can be expressed only upon exposure of the integrated DNA construct to a Cre recombinase. Homology arms ("2.4 kb Rosa HA 5'" and "2.8 kb Rosa HA 3"') to the mouse Gt(ROSA)26Sor locus are shown flanking the construct. The construct includes, from 5' to 3' with respect to the transcribed RNA, an intronic branch point, polypyrimidine stretch, and 3' splice site (labeled "splice acceptor"), a IoxP site, an EM7 promoter, a neomycin phosphotransferase coding sequence (neo^{r}), a signal for transcription termination and mRNA polyadenylation from the mouse Pgk1 gene (PGKp(A)), followed by a IoxP site, a sequence that codes for the diphtheria toxin A fragment (DTA), followed by an internal ribosome entry site (IRES) and a sequence that codes enhanced green fluorescent protein (eGFP), followed by a transcription termination and mRNA polyadenylation signal from the gene for human β-globin (β-gl p(A)). Sequence lengths are not drawn to scale.

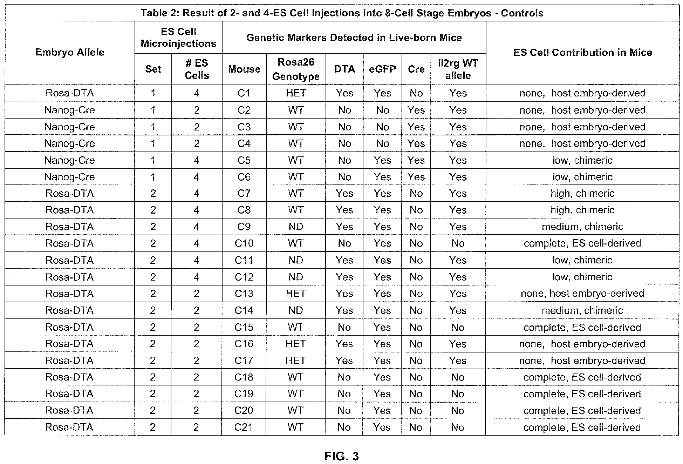

FIG. 3 shows genotyping results for control groups (two sets) that reflect microinjections of 2 and 4 donor mouse ES cells into 8-cell stage mouse embryos. The alleles present in the host embryo are either Rosa-DTA (Rosa26-IoxP-neo-poly(A)-Ioxp-DTA-IRES-eGFP, targeted allele at the Rosa26 locus) or Nanog-Cre (Nanog-Cre-poly(A)-PGKp-neo-poly(A), a random insertion allele of a Nanog-modified BAC); the microinjected ES cell carries a reverse COIN conditional allele (eGFP-poly(A)-hUbCp-neo-poly(A), inserted at the X-linked II2rg gene). "Mouse" refers to an arbitrary designation of an individual mouse.

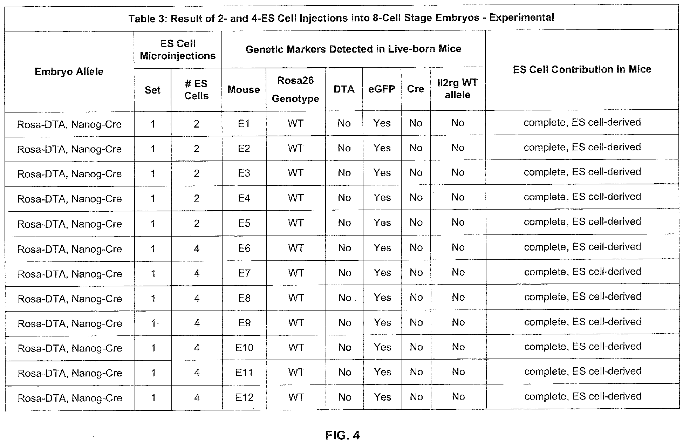

FIG. 4 shows genotyping results for an experimental group (one set) that reflect microinjections of 2 and 4 donor mouse ES cells into 8-cell stage mouse embryos. The host embryos carry both the Rosa-DTA and the Nanog-Cre alleles.

### DETAILED DESCRIPTION

The invention in its broadest sense is as defined in the independent claims.

Before the present compositions and methods are described, it is to be understood that the invention is not limited to particular methods and experimental conditions described, as such methods and conditions may vary. It is also to be understood that the terminology used herein is for the purpose of describing particular embodiments only, and is not intended to be limiting, since the scope of the present invention will be limited only by the appended claims.

Unless defined otherwise, all technical and scientific terms used herein include the same meaning(s) as commonly understood by one of ordinary skill in the art to which this invention belongs. Although any methods and materials similar or equivalent to those described herein can be used in the practice or testing of the present invention, specific methods and materials are described.

Genetically modified animals are described herein, as well as non-human embryos derived in whole or in part from non-human donor cells. Methods and compositions are described herein for making a non-human embryo or a non-human animal from a host embryo and a donor cell.

The methods include introducing a non-human donor cell into a non-human host embryo, growing the host embryo in an animal under conditions suitable for gestation, and obtaining an embryo or an animal having a genetic contribution from the donor cell. In various aspects, the method includes making animals that are substantially or wholly derived from the donor cell (for example, an ES cell) by introducing the donor cell into an embryo (for example, a pre-morula, morula, or blastocyst). Although various aspects can be practiced using tetraploid embryo technology, the methods described herein do not require using tetraploid embryo technology. Although various aspects can be practiced employing 4- and 8-cell stage embryos, the methods described herein also include methods for introducing donor cells at other stages, for example, at a blastocyst stage.

Methods and compositions are also described herein for making mice or embryos that are substantially or wholly derived from the donor cell by introducing the donor cell into an embryo that is, for example, a two-cell stage embryo, a four-cell stage embryo, an 8-cell stage embryo, a 16-cell stage embryo, a premorula, a morula, an uncompacted morula, a compacted morula, a blastocyst, lacking or substantially lacking a primitive endoderm, or a blastocyst comprising a primitive endoderm

Methods and compositions are described herein for making non-human host embryos lacking a viable ICM or an ICM capable of developing into an embryo or a live-born animal, or for making non-human host embryos that comprise an ICM that is incapable of proliferating, and for using such embryos as hosts to make non-human embryos or animals derived in full or in part from a donor cell by introducing the donor cell into the host embryo and growing the embryo under conditions suitable for development of the embryo.

Although the present disclosure can also be employed with tetraploid embryo technology, or with a method comprising introducing the donor cells at a pre-morula stage, the methods are suitable, for example, for use with donor cells and embryos without employing tetraploid fusions and at a pre-morula, morula or post-morula stage such as, for example, in the blastocyst stage. Suitable donor cells include, for example, ES cells. Suitable donor cells also include PS cells, for example iPS cells, and any other suitable cell that is capable of populating an embryo.

The methods include employing a host embryo that is modified (physically, chemically, or genetically) to reduce the ability of cells of the host embryo's ICM to proliferate or populate the embryo. In various aspects, the host embryo's ICM becomes effectively unable to contribute to the development of the embryo. This affords donor cells a competitive advantage in populating the host embryo, even when the donor cell is introduced at a relatively late stage (e.g., at a morula, post-morula, or blastocyst stage). In various aspects, methods and compositions described herein for making a mouse that is substantially or fully derived from the donor cell (i.e., with little or no contribution from host cells) in the F0 generation. In various aspects, a mouse that is substantially or fully derived from a donor cell is made, without employing tetraploid embryo technology and without the need for introducing donor cells at a pre-morula stage.

In various aspects, an ICM cell is "substantially incapable of proliferating" or an ICM cell that is reduced in its ability to proliferate or populate an embryo is an ICM cell that is genetically modified so as to be incapable of effectively competing with a donor cell to populate an embryo or to contribute to the tissues of an animal that develops from the embryo. In various aspects, such ICM cells include those that are unable to survive for a sufficient amount of time to reproduce, are unable to reproduce at a speed that allows host-derived ICM cells to effectively compete with a donor cell, or are modified so as to conditionally express a gene toxic to the cell (e.g., a gene encoding a toxic RNA or toxic protein) that kills the host-derived ICM cell at a point of development of the embryo wherein death of cells of host ICM lineage would not inevitably lead to death or expiration or re-absorption of an embryo into which viable donor cells capable of populating the embryo are introduced.

Early stage mouse embryos, such as blastocysts (e.g., a 64-cell stage embryo) comprise two main types of cells-cells forming a trophectoderm and cells forming the ICM. The trophectoderm is an essentially hollow ball-like structure that provides protection and support to the cells of the ICM, which are contained by the trophectoderm.

During early mouse embryo development, cells of the ICM are associated with a fluid-filled cavity (a blastocoel) also contained by the trophectoderm. The cells of the trophectoderm do not ultimately give rise to components of the resulting mouse, and form instead extraembryonic tissues (e.g., placenta and yolk sac). In contrast, the ICM cells within the trophectoderm give rise to all cell types and tissues of the resulting mouse. The methods and compositions described herein include methods and compositions that exploit this difference in mouse embryo composition, and in various aspects are directed to ablating a host embryo's ICM cells in whole or in part, and introducing a donor cell to populate the host embryo to develop an animal that is derived from the donor cell, including animals derived in whole or in substantial part from the donor cell. In a specific aspect, all or substantially all of the cells of the resulting animal are donor cell-derived. In various aspects, the donor cell comprises a genetic modification, for example, a mutation, deletion, or insertion of a gene or gene fragment, and is homozygous or heterozygous for the modification.

Without limitation of the claims by theory, one view is that at least some cells of an ICM can contribute to formation of a primitive endoderm in a blastocyst, as well as to the epiblast. Under this view, proteins or genes that are toxic to host cells (e.g., toxic to host embryo ICM), could result in destruction of the primitive endoderm of the blastocyst before donor cells have proliferated and/or differentiated sufficiently to contribute to the primitive endoderm. Under this view, it would be advantageous when employing a blastocyst as a host embryo to select a blastocyst at a stage where the primitive endoderm has not yet formed, or is in the process of forming, in order to introduce the desired donor cells (e.g., the ES cells or pluripotent cells). Under this view, the donor cells would have an opportunity to participate in the formation of primitive endoderm and increase the chances of the resulting embryo to reach term. Thus, in some aspects, the donor cells are introduced at a stage prior to the formation of the primitive endoderm, or at a stage prior to substantial formation of the primitive endoderm.

Methods and compositions in accordance with the present disclosure are included in the discussion of specific aspects below, which are provided as examples. In the discussion below, two genetically modified mice are made: (1) a first activator mouse, comprising a gene encoding an activator (in a specific aspect, a recombinase) whose expression is driven by a developmentally-regulated promoter (in a specific aspect, a promoter that drives expression of a gene at a stage when the Nanog promoter drives expression, i.e., at a stage when the Nanog gene is transcribed), and (2) a second genetically modified mouse (a responder mouse) comprising a gene that is toxic to a cell (e.g, a gene encoding a protein toxic to a cell; in a specific aspect, a DTA gene) in a construct placed at an expression-permissive locus (in a specific aspect, the Gt(ROSA)26Sor locus), wherein the toxic gene is not expressed until a sequence located between the toxic gene's coding sequence and the promoter and that prevents expression of the toxic gene (e.g., an intervening nucleic acid sequence; in a specific aspect, a floxed neo^{r}-gene-poly(A) cassette) is acted on by the activator (e.g., by action of a site-specific recombinase; in a specific embodiment, Cre).

Mice made according to the paragraph above (e.g., an activator mouse and a responder mouse) are then mated. If both the responder mouse and the activator mouse are homozygous for their respective alleles, the mating will result in no live offspring. If the mice are each heterozygous, then only embryos having both alleles (i.e., activator and responder alleles) will not survive. Expression of the activator of the first genetically modified mouse (the activator mouse) will activate the toxic gene of the second genetically modified mouse (the responder mouse). The activator will be expressed, and activation will occur, and it will occur at a point in embryo development that corresponds with the switching on of the developmentally-regulated promoter. The expressed activator will act on the sequence that prevents expression of the toxic gene, thus allowing the toxic gene to become expressed.

Upon expression of the toxic gene, the cells of the ICM will fail to thrive, fail to proliferate, fail to be able to compete effectively with donor cells, and/or die as the ultimate result of the expression of the toxic gene. Because the ICM cells cannot thrive, proliferate, compete with donor cells, and/or survive, no offspring derived from the host embryo's ICM will form. Instead, donor cells (in a specific aspect, mouse donor ES cells; comprising a genetic modification, if desired) that are introduced into the embryo, wherein the donor cells lack the activator and/or responder (e.g., they lack the toxic gene and/or or ability to express it), can be introduced to populate the host embryo. Since the donor cells do not make the toxic gene, the donor cells are capable of populating the embryo and developing into a more advanced embryo and, in the appropriate circumstances, a mouse offspring. In some aspects, the mouse offspring is fully derived from the donor cell, and may comprise any desirable genetic modification, where the mouse may be, e.g., heterozygous or homozygous for the genetic modification, as desired.

The genetically modified mouse comprising the gene for the activator driven by the developmentally-regulated promoter can be made by placing an activator gene (with or without a promoter) at any suitable locus in the genome by any method known to those of ordinary skill. Placement of the nucleic acid construct comprising the activator gene can be made by any suitable method, for example, homologous recombination or random integration. The present disclosure is not limited by any particular method for introducing the activator gene, and is not limited to placement at any specific locus.

Similarly, a genetically modified mouse comprising a responder gene in, e.g., a promoterless construct placed at an expression-permissive locus can be made, for example, by introducing a nucleic acid construct comprising the responder gene (e.g., a DTA coding sequence) and any sequence that prevents expression of the responder gene (e.g., a marker and/or transcription termination signal flanked on both sides by site-specific recombinase recognition sites) into a genome at any suitable locus in the genome by any method known to those of ordinary skill.

The present disclosure is not limited by any particular method for introducing the construct comprising the responder gene, and is not limited to placing the construct comprising the responder gene at any specific locus. Although examples discussed herein employ a targeting construct comprising homology arms corresponding to the Gt(ROSA)26Sor locus, integration can proceed by either homologous recombination to a targeted site (e.g., the Gt(ROSA)26Sor locus), or by random integration to any suitable expression-permissive locus. All that is required is that, upon expression of the activator gene (e.g., expression of a recombinase), the toxic gene of the responder is expressed.

An expression-permissive locus in accordance with the present disclosure refers to a locus within a genome that does not interfere with expression of the responder gene. An expression-permissive locus includes a locus that is selected by employing a construct that randomly inserts the responder gene into the genome. For example, a construct comprising the responder gene and a marker gene adjacent to the responder gene is introduced into a genome, and expression of the marker (e.g., a drug resistance gene) is an indication that integration has occurred at an expression-permissive locus.

In another example, the responder gene can be introduced in a construct that contains sequence arms that are homologous to a pre-selected or specific locus that is known or suspected to be expression-permissive (e.g., a construct comprising a toxic gene and homology arms directing the construct to the pre-selected or specific locus).

In various aspects, selection of the expression-permissive locus can be facilitated in some cases by including a promoterless marker (i.e., lacking a promoter that is active in the cell type in which the construct is placed) in the construct comprising the responder gene (e.g., adjacent to the responder gene). In such an instance, cells can be screened by observing expression of the marker, ensuring that the locus is permissive with respect to expression of the marker. In various aspects, removal of the marker (and any sequence(s), such as, for example, a transcription termination sequence following the marker) can be an event that allows the expression-permissive locus to drive expression of the responder gene, since in such an aspect the responder gene upon excision of the marker and any associated sequence(s) (e.g., a transcription termination sequence), the responder gene becomes operably linked to a promoter. In various aspects, the promoter can be present in a homology arm that directs a construct containing the responder gene to a specific or pre-selected locus. In various embodiments, sequence encoding a splice acceptor site can be employed in the responder construct, located, e.g., 5' with respect to the toxic gene transcript, to assist in operably linking the toxic gene to a promoter upon, e.g., excision of the marker gene and any associated sequence(s).

In various aspects, a responder gene can be introduced into a genome using a construct that comprises a sequence that is capable of driving expression of the responder gene upon expression of an activator gene. In a specific aspect, a promoter is placed 5' with respect to a marker gene's transcript, the marker is followed by a transcription termination sequence, which is followed by the responder gene. In these aspects, the construct comprising the responder gene can be placed anywhere in a genome that the promoter is not silenced. Here, the expression-permissive locus is a locus that does not silence the promoter of the construct comprising the responder gene.

For example, the toxic gene can be placed in the genome in a nucleic acid construct that comprises a promoter operably linked to, for example, a IoxP-marker-poly(A)-IoxP sequence. In such a situation, the promoter is introduced into the genome with the toxic gene and an intervening sequence (loxP-marker-poly(A)-loxP) between the toxic gene and the promoter, wherein the intervening sequence prevents expression of the toxic gene until acted upon by a Cre recombinase. Once acted upon by the Cre recombinase, the IoxP-marker-poly(A)-IoxP intervening sequence is excised, placing the promoter in operable linkage with the toxic gene, enabling expression of the toxic gene. In such an aspect, the locus is expression-permissive because it does not silence expression of the marker gene before the action of the recombinase, and does not silence expression of the toxic gene once the toxic gene becomes operably linked to the promoter by the action of the recombinase.

The developmentally-regulated promoter that drives activator gene expression includes a promoter selected such that it is not active, i.e., that it does not drive expression of a gene operably linked to it, until host embryo development is at a desired (or pre-selected) stage. In various aspects, the promoter is not active in the trophectoderm (e.g., it is a promoter for a gene that is not transcribed in the trophectoderm under natural conditions in a normal mouse embryo), and the development stage is one in which the trophectoderm is present but prior to gastrulation (e.g., blastocyst stage), or in a pre-blastocyst stage. In this way, and in these aspects, for example, the activator that is operably linked to the promoter is expressed no earlier than the morula stage, but is expressed no later than gastrulation. Thus in one aspect, a "developmentally-regulated" promoter of the activator gene includes a promoter that drives expression of a gene operably linked to it no earlier than the morula stage. In another aspect, a "developmentally-regulated" activator gene drives expression no earlier than the morula stage and does not drive expression at and/or after gastrulation. In the specific examples provided, a promoter of a mouse Nanog gene is contained in the DNA construct introducing the activator gene (here, encoding a Cre recombinase gene) into a genome to make an activator mouse, although any promoter that has, or can be modified to have, the discussed properties is suitable.

It should be noted that in the Examples provided herein, the DNA construct containing the Cre gene flanked on both sides by Nanog sequences (see Fig. 1) does not necessarily have to insert at a Nanog locus in the mouse genome for the embodiment of the invention to function as desired. In the Examples provided herein, both Nanog alleles that naturally occur in the wild-type mouse were present as measured by quantitative PCR (data not shown) in the mouse genetically modified by the DNA construct of Fig. 1, as well as the Nanog introduced by the DNA construct of Fig. 1.

In various aspects, the developmentally-regulated promoter is a promoter active only in early-stage embryogenesis (e.g., is a promoter for a gene transcribed only in early-stage embryogenesis), such as in morula and blastocyst stage embryos (Chambers et al. (2003) Cell, 113:643-655), and is expressed generally only in the cells of the ICM. A developmentally-regulated promoter is employed so that an activator gene to which the promoter may be operably linked (e.g., where the activator gene encodes a recombinase, e.g., Cre, Flp, etc.) is expressed only when such a developmentally-regulated promoter is active, i.e., during early embryo development, and, e.g., especially when the ICM is being formed.

One advantage of the Nanog promoter is that it is not active in the trophectoderm, which is advantageous in that the constructs described herein do not lead to the death of trophectoderm cells by expression of the activator gene (e.g., Cre) in trophectoderm cells and concomitant expression of the responder gene (e.g., due to excision of a sequence adjacent to the responder gene, e.g., a DTA gene, which prevents expression of the responder).

In various aspects, the activator comprises a protein that is capable of activating a promoter such that a gene operably linked to the promoter is capable of expression from the promoter in the presence of the activating protein. In various aspects, the activator protein comprises a modified repressor, wherein the modified repressor is capable of activating expression from a promoter, such as, for example, the modified tet repressor operating in a tet on/off expression system. Other aspects include a Dox-dependent system and a rapamycin-dependent system.

In various aspects, the activator gene encodes a site-specific recombinase. The recombinase may be a recombinase that is not naturally expressed in the cell or embryo. In various aspects, a recombinase that is not active in the normal wild-type mouse at the stage of development in which the developmentally regulated promoter drives expression, or is absent from a wild-type mouse, is employed.

In various aspects, where the activator gene encodes a site-specific recombinase, any suitable site-specific recombination recognition sites can be employed to flank (e.g., on both sides) a sequence that prevents expression of the responder gene. The site-specific recombinase site may be a IoxP site, or variants thereof, (recognized by Cre recombinase or a modified Cre recombinase), a FRT site, or variants thereof, (recognized by Flp recombinase or a modified Flp recombinase), or any other suitable recombinase recognition site. If the recombinase recognition sites are placed in the same orientation as defined by their asymmetric core region, intervening sequences (i.e., sequences located between the recombinase recognition sites) are excised after exposure to the appropriate recombinase. If the recombinase recognition sites are placed in the opposite orientation with respect to one another as defined by their asymmetric core region, the intervening sequences are inverted after exposure to the appropriate recombinase.

A variety of markers can be used with the methods and compositions described herein. Suitable markers include selectable markers that operate both in conjunction with a protector cassette and as a selectable marker to identify integration events of the construct into the genome of the donor cell. Selectable markers may be any marker known to the art, including, but not limited to, a drug resistance gene, such as a gene for, for example, neomycin phosphotransferase (neo^{r}), hygromycin B phosphotransferase (hyg^{r}), puromycin-N-acetyltransferase (puro^{r}), blasticidin S deaminase (bsr^{r}), xanthine/guanine phosphoribosyl transferase (gpt), Herpes simplex virus thymidine kinase (HSV-tk) and fusions of tk with neo^{r}, hyg^{r} or puro^{r}, or reporter genes, such as, for example, cyan fluorescent protein (CFP), green fluorescent protein (GFP), enhanced GFP (eGFP), yellow fluorescent protein (YFP), enhanced YFP (eYFP), blue fluorescent protein (BFP), enhanced BFP (eBFP), red fluorescent protein from the Discosoma coral (DsRed), MmGFP (Zernicka-Goetz et al. (1997) Development 124:1133-1137) or others familiar to those of ordinary skill. Suitable selection agents for drug resistance genes include G418 (with neo^{r}), puromycin (with puro^{r}), hygromycin B (with hyg^{r}), blasticidin S (with bsr^{r}), mycophenolic acid and 6-thio(guanine) (with gpt) and gancyclovir or 1(2'-deoxy-2'-fluoro-beta-D-arabinofuranosyl)-5-iodouracil (FIAU) (with HSV-tk).

The toxic gene can be any nucleotide sequence encoding a product that either alone or in combination with another agent leads to the failure to thrive, failure to proliferate, failure of host ICM cells to compete with donor cells, or death of the cell expressing the toxic gene. A toxic gene can encode a protein (e.g., can be a cDNA encoding a toxin), or can encode a microRNA that is deleterious to the ICM. Preferred toxic genes include, but are not limited to, the genes for DTA (Matsumura et al. (2004) Biochem. Biophys. Res. Commun. 321:275-279), attenuated DTA, tox-176 (Drago et al. (1998) J Neuroscience 18:9845-9857), herpes simplex virus 1 thymidine kinase (HSV-tk), PE40 (Saito et al. (1994) Cancer Res. 54:1059-1064), ricin and any other suitable toxic gene known to those of ordinary skill. Toxically-effective fragments of toxic genes are also suitable. In various aspects, the toxic gene should not substantially lead to the failure to thrive, failure to proliferate, or death of cells that do not express the toxic gene.

### A Genetically Modified Mouse Having a Recombinase Operably Linked to a Developmentally Regulated Promoter

A genetically modified mouse having an activator gene (e.g., a gene encoding a recombinase) that was inserted into the mouse genome using a DNA construct having sequences of a developmentally-regulated promoter, and is expressed in a developmentally-regulated manner (e.g., as Nanog is expressed) was employed as an activator strain. Such an activator strain having a gene encoding an activator protein (in the example, a Cre recombinase) that is expressed in a developmentally-regulated manner was made and used to breed with a second mouse strain, a responder strain (described below). The strain having the activator was designated the "Nanog-Cre Tg" strain.

The Nanog-Cre Tg strain was made as described in Example 1. A DNA construct comprising Nanog promoter sequences and a Cre recombinase gene was made and introduced into a mouse ES cell, which was used as described to make a homozygous Nanog-Cre mouse. the DNA construct contained sequences from the Nanog gene encoding the complete 5'UTR and part of the 3'UTR as described in Example 1, and the sequence encoding the 5'UTR was followed by the sequence encoding the Nanog protein from the first methionine codon to the second in-frame methionine codon (indicated as "2nd ATG" in the DNA sequence diagramed in FIG. 1). Linked to the Nanog protein coding sequence was a sequence encoding the Cre recombinase with a nuclear localization signal. The construct also contained a FRT-flanked (flanked on both sides) neomycin resistance marker cassette containing a promoter for expression and G418 selection in eukaryotes (PGK promoter) and a promoter for expression and selection in prokaryotes (EM7 promoter) and a sequence encoding a transcription termination and polyadenylation signal.

As described in the examples, the Nanog-Cre Tg mouse produced viable mice upon breeding that were homozygous for the Nanog-Cre modification, effectively establishing a strain of mouse having a recombinase gene (i.e., an activator gene) expressed in a developmentally-regulated manner (i.e., in the embryo at or during ICM formation) and in the developing genital ridges. Quantitative PCR demonstrated that both native alleles of the Nanog gene were present (data not shown) and a single copy o fthe Nanog-Cre construct had inserted elsewhere into the genome. Nevertheless, the recombinase was expressed in a manner consistent with the developmental pattern of Nanog expression, as measured by expression of Cre driven by the Nanog promoter in ES cells.

### A Genetically Modified Mouse Having a Toxic Gene at an Expression-Permissive Locus

A genetically modified mouse having a toxic gene at expression-permissive locus, wherein expression of the toxic gene requires expression of an activator gene, was employed as a responder strain. The toxic gene is not expressed in the responder strain, because the responder strain does not carry an activator gene. Such a responder strain was made, having a gene encoding a toxic protein (in the example, DTA) preceded by a sequence that prevents its expression in the absence of an activator protein (in the example, a floxed neo^{r}-poly(A) sequence), the construct flanked by homology arms to an expression-permissive locus (in the example a Gt(ROSA)26Sor locus). The responder strain was designated the "ROSA-floxed-STOP-DTA" strain.

The ROSA-floxed-STOP-DTA strain was made as described in the examples. A DNA construct comprising a floxed stuffer sequence (a floxed neo^{r}-poly(A) sequence) adjacent to a DTA coding sequence was made having homology arms to the mouse Gt(ROSA)26Sor locus and introduced into a mouse ES cell, which was used as described to make a homozygous ROSA-floxed-STOP-DTA mouse. As shown in FIG. 2, the DNA construct contained, from 5' to 3', with respect to the Gt(ROSA)26Sor transcript, a 5' homology arm with homology to 2.4 kb of the mouse Gt(ROSA)26Sor locus, a splice acceptor sequence, a IoxP site, an EM7 promoter, a sequence encoding neomycin phosphotransferase followed by a PGK poly(A) signal, a IoxP site, a sequence encoding DTA followed by an IRES, a sequence encoding eGFP followed by a β-globin poly(A) signal, and finally a 3' homology arm with homology to 2.8 kb of the Gt(ROSA)26Sor locus. As described in the examples, the responder mouse produced viable mice upon breeding that were homozygous for the DTA gene, effectively establishing a responder strain of mouse having a conditional toxic gene at an expression-permissive locus, with expression of the toxic gene conditioned upon the presence of an activator protein (here, a recombinase) specific for removing a sequence that prevents expression of the toxic gene (floxed neo^{r}-poly(A)) positioned adjacent to the toxic gene or between the toxic protein coding sequence and the Gt(ROSA)26Sor promoter.

### Breeding the Developmentally Regulated Cre Strain with the Conditional Lethal Strain

The developmentally-regulated activator strain (i.e., the Nanog-Cre Tg mouse) was mated with the responder mouse (i.e., the ROSA-floxed-STOP-DTA mouse). Mice were mated as described in Example 3. No matings of the Nanog-Cre Tg mouse and the ROSA-floxed-STOP-DTA mouse resulted in offspring. This result is consistent with the mating producing an embryo having both a Nanog-driven Cre (or a Cre driven by a similarly developmentally-regulated promoter to Nanog promoter) and the DTA construct with the floxed sequence preventing its expression in the absence of Cre, and expression of Cre when Nanog (or a similarly developmentally-regulated gene) is turned on in the embryo would result in excision of the floxed sequence adjacent to the DTA sequence and consequent expression of the DTA gene. Expression of the DTA gene would result in the failure of the ICM of the embryo to survive, and no live births would result in the absence of a donor cell added to the embryo.

Specific examples are provided in what follows. In general and overall, about 703 mice were bred (about 141 C57BL/6 background and about 562 Swiss Webster background), without the occurrence of a live-born mouse that was double heterozygous for Nanog-Cre and ROSA-floxed-STOP-DTA.

### Introducing Donor ES Cells into an Embryo Made from a Cross of the Developmentally Regulated Cre Strain and the Conditional Lethal Strain

The developmentally-regulated activator strain (i.e., the Nanog-Cre mouse) was mated with the responder mouse having the conditional lethal toxic gene (i.e., the ROSA-floxed-STOP-DTA mouse). Mice were mated as described in the examples, and embryos from the pregnant female were harvested and designated host embryos for receipt of donor ES cells, as described in the examples.

### EXAMPLES

The following examples are included so as to provide those of ordinary skill in the art with a disclosure and description of how to make and use methods and compositions described herein, and are not intended to limit the scope of what the inventors regard as their invention. Efforts have been made to ensure accuracy with respect to numbers used (e.g., amounts, temperature, etc.) but some experimental errors and deviations should be accounted for. Unless indicated otherwise, parts are parts by weight, molecular weight is average molecular weight, temperature is in degrees Centigrade, and pressure is at or near atmospheric.

### Example 1: Construction of a Nanog-Cre Mouse Strain

The Nanog-Cre construct for the Nanog-Cre (activator) strain (designated "Nanog-Cre Tg") was made by bacterial homologous recombination (BHR) using the bacterial artificial chromosome (BAC) 359m22 (Incyte Genomics 129/SvJ library) as the source of the mouse Nanog gene and as recipient of the inserted Cre gene. The protein coding sequence of the bacteriophage P1 Cre recombinase protein (Abremski and Hoess (1984) J. Biol. Chem. 259:1509-1514) was inserted into the Nanog gene in such a manner that the second in-frame AUG codon of Nanog mRNA becomes the first codon of Cre. The coding region of the Nanog gene spanning 6.3 kb was replaced by a 3.4 kb cassette containing the in-frame Cre and the neo^{r} gene. The neo^{r} gene was placed under the control of the mouse phosphoglycerate kinase I (PGK) (Pham et al. (1996) Proc. Natl. Acad. Sci. USA 93:13090-13095) and bacterial EM7 promoters (for positive selection in eukaryotic and prokaryotic cells, respectively) and was followed by the poly(A) signal of the PGK gene. The flanking FRT sites allow the removal of the PGK-EM7-neo^{r} cassette in the presence of Flp recombinase (Joyner, A. L. 1999 Gene Targeting; a Practical Approach. Oxford University Press Inc., Oxford, New York).

The Nanog-Cre transgenic line (i.e., Nanog-Cre Tg) was generated by random insertion of the Nanog-Cre construct into hybrid ES cells (designated VGF1;
129SvEvTac/C57BL6NTacF1 cells; see Valenzuela et al. (2003) Nature Biotech. 21:652-659, infra). A clone (designated 1297A-D5) that carried a single copy of the Nanog-Cre transgene was microinjected into an 8-cell stage embryo according to the method of Poueymirou et al. (2007) Nature Biotech. 25:91-99 (the "VelociMouse®" method) to produce Nanog-Cre transgenic mice, which were bred to homozygosity as determined by quantitative PCR of the Cre gene.

### Example 2: Construction of a Conditional Lethal Mouse Strain Having a DTA Toxic Gene

A conditional lethal mouse strain (designated "ROSA-floxed-STOP-DTA") was made by introducing the construct shown in FIG. 2 (containing the DTA gene adjacent to a neo^{r} marker and poly(A) signal flanked on both sides by IoxP sites) into a CJ7 mouse ES cell.

Briefly, DTA coding sequence was inserted downstream of a floxed cassette containing a neo^{r} coding sequence and four tandem polyadenylation signals (Soriano, P. (1999) Nat. Genet. 21:70-71). The DTA coding sequence is followed by an IRES-eGFP sequence and four β-globin poly(A) sequences. A 2.4 kb segment upstream of the Nhe I site in intron 1 of the Gt(ROSA)26Sor locus, and a 2.8 kb segment downstream of the Nhe I site in intron 1 of Gt(ROSA)26Sor locus, were employed as arms for targeted homologous recombination in the CJ7 ES cells.

Although homology arms corresponding to the Gt(ROSA)26Sor locus were employed, the efficacy described herein does not rely on employing any particular homology arms and does not rely on insertion at any particular locus; all that is required is that the "floxed-STOP-toxic gene" be placed at a locus where the DTA can be expressed (e.g., by a promoter already in the genome or by a promoter knocked in along with the DTA construct) upon excision of the floxed-STOP cassette adjacent to the DTA coding sequence.

The DTA coding sequence is not expressed until excision of the neo^{r} gene's poly(A) by Cre recombinase. Thereafter, a splice acceptor upstream of the DTA coding sequence will facilitate its expression from the expression-permissive locus. The CJ7 mouse ES cell bearing the ROSA-floxed-STOP-DTA modification at the Gt(ROSA)26Sor locus (FIG. 2) was injected into a blastocyst derived from the mating of C57BL/6 mice. Mice heterozygous for the Rosa-floxed-STOP-DTA were bred to homozygosity.

### Example 3: Breeding the Nanog-Cre Strain with the Conditional Lethal Strain

Homozygous Nanog-Cre mice were mated with heterozygous ROSA-floxed-STOP-DTA mice, and heterozygous Nanog-Cre mice were mated with heterozygous ROSA-floxed-STOP-DTA, generating 141 live offspring. Of the 141 live offspring, none were found to be double heterozygotes (i.e., none were found to be heterozygous for both Nanog-Cre and ROSA-floxed-STOP-DTA).

### Example 4: ES-Cell-Derived Mice From Blastocyst Injections

Homozygous Nanog-Cre Tg mice were mated with homozygous ROSA-floxed-STOP-DTA mice to generate embryos suitable for introducing donor ES cells.

ES cells that were genetically different from the host embryos were employed to readily determine the level of chimerism in any animals born from the cross. Donor ES cells were VGF1-derived and contained a conditional allele of the II2rg gene (or the gene for IL-2Ry) (clone 1371A-G6), which allowed for genotyping of mice according to the characteristics shown in Table 1:

| **Table 1 Genotypes of ES Cells and Host Embryos** | | |
|---|---|---|
| **GENE** | **EMBRYO** | **ES CELL** |
| **Cre** | + (present) | - (absent) |
| **DTA** | + (present) | -- (absent) |
| **II2rg** | + (wild-type allele present) | - (wild type allele absent)* |
| *The mutated II2rg gene is on the single X chromosome of the male ES cell. | | |

To generate 8-cell stage embryos (positive control group) and blastocyst stage embryos for donor ES cell injection, 33 homozygous Nanog-Cre Tg females were super-ovulated and mated with homozygous Rosa-floxed-STOP-DTA males. One hundred and forty 8-cell stage embryos were harvested from 25 plugged females.

Twenty-five of the 140 harvested 8-cell stage embryos were injected with VGF1 clone 1371A-G6 (donor ES cell) according to the VelociMouse® method, as described. The remaining 115 embryos were cultured overnight to the blastocyst stage in KSOM. After overnight culture, 33 blastocyts were suitable for injection. The remaining 82 were viable but had only started to cavitate. Thirty-nine of these late morula/early blastocyst embryos were transferred un-injected into pseudo-pregnant mothers to serve as negative controls, and the remaining 43 were discarded. The 33 blastocyst-stage embryos were injected with VGF1 clone 1371A-G6 and cultured in KSOM prior to transfer in accordance with standard procedures known in the art (Hogan et al., Manipulating the Mouse Embryo, Cold Spring Harbor Laboratory Press, 2d Ed., 1994).

### Example 5: Genotyping Results for ES Cell-Derived Mice From Blastocyst Injections

Genetic analysis for the markers listed in Table 1 of tail biopsies obtained from the F0 generation mice failed to detect host embryo-specific markers, i.e., the Cre gene and the wild type II2rg allele, and indicated a single copy of the neo^{r} gene, demonstrating that mice were derived from the donor ES cells.

The 25 8-cell stage embryos injected with the 1371A-G6 clone using the VelociMouse® method produced three live mice. As expected, the 39 uninjected blastocysts produced no live offspring. In contrast, the 33 blastocysts injected with donor ES cells produced six live born mice, demonstrating that the donor ES cells could rescue the lethal effects of the combination of Nanog-Cre and Rosa-floxed-STOP-DTA in the host embryos.

### Example 6: Donor Cell-derived Mice by Injecting 2 or 4 ES Cells

Mice wholly derived from donor ES cells were made using the embryos carrying both the Nanog-Cre and Rosa-floxed-STOP-DTA constructs, under conditions that reduce the likelihood of producing wholly ES cell-derived mice. Injecting 2 or 4 donor cells into an 8-cell stage embryo is less likely to result in wholly ES cell-derived mice than injection of more cells, e.g., 6-8 or more cells. Briefly, the VelociMouse® method, described above, was used to make host embryos by injecting either 2 or 4 or 8 donor mouse ES cells into embryos (8-cell stage embryos) carrying either (1) the Rosa-floxed-STOP-DTA construct alone (control), the Nanog-Cre construct alone (control), or (2) both the Nanog-Cre and Rosa-floxed-STOP-DTA constructs (experimental) described above. Donor ES cells were as described in Example 4, i.e., VGF1-derived (129Sv/EvB6 F1) and contained a conditional allele of the II2rg gene. The strain background for host embryos was a mixed strain of C57BL/6 and 129 (predominantly C57BL/6).

Briefly, sires carrying the ROSA-floxed-STOP-DTA construct (homozygous or heterozygous for the ROSA-floxed-STOP-DTA construct) were mated with either wild type B6 females (control) or with females carrying the Nanog-Cre construct (homozygous for the Nanog-Cre construct). Matings (and resulting genotyping) were done in two sets beginning on different dates. Microinjections and genotyping were also done in different sets on different dates. In total, 36 pups were born and all live born pups were genotyped for contribution from each parent and from the donor ES cells. Genotyping was performed using TaqMan™ assays for the presence or absence of markers for the male (DTA and eGFP; ROSA was measured by a loss of allele assay for the Gt(ROSA)26Sor gene), the female (CRE), and for the donor ES CELL (II2rg and eGFP) in order to unambiguously identify the genotypes of the pups. Results are shown in Table 2 for controls and Table 3 for experimentals; sets of microinjections are designated "1," and "2." Criteria for determining complete ES cell contribution in pups were the same as those described in Poueymirou et al. (2008) Nature Biotech. 25(1):93-99 at Fig. 2a. The limit of detection for II2rg is about less than 0.1% (host contribution detectable to below 0.1%; see Fig. 2a of Poueymirou et al.). Genotyping was conducted on the following tissues: brain, lung, liver, spleen, heart, skin, hind limb, fore limb, stomach, kidney, intestine, tail.

As shown in FIG. 3 (Table 2) and FIG. 4 (Table 3), where the genotype of an embryo includes both the Nanog-Cre and the the ROSA-floxed-STOP-DTA constructs, only completely ES cell-derived pups are generated. The data indicate that when fewer ES cells are injected (e.g., 2 or 4 ES cells) into an embryo lacking the Nanog-Cre and the the ROSA-floxed-STOP-DTA constructs, chimerism is shown.

## Claims

1. A mouse embryo, comprising a cell having in its genome:
(a) a site-specific recombinase gene operably linked to a Nanog promoter that expresses the site-specific recombinase gene in a cell of the inner cell mass (ICM) but not the trophectoderm;
(b) a gene encoding a toxin, operably linked to a promoter, wherein the toxin gene is present at an expression-permissive locus; and
(c) a nucleic acid sequence that prevents expression of the gene encoding the toxin, wherein the nucleic acid sequence is located between the promoter for the gene encoding the toxin and the coding sequence of the gene encoding the toxin and is flanked on both sides by recombinase recognition sites such that in the presence of the site-specific recombinase the nucleotide sequence is removed and the promoter for the gene encoding the toxin drives transcription of the gene encoding the toxin.

2. A method for making a mouse or mouse embryo from a mouse donor cell and a host embryo, comprising:
(a) introducing mouse donor cells into a mouse host embryo, wherein the mouse donor cells are selected from the group consisting of embryonic stem (ES) cells, pluripotent stem (PS) cells, or induced pluripotent stem (iPS) cells, and wherein the host embryo is a pre-morula stage embryo and comprises
(i) a site-specific recombinase gene operably linked to a Nanog promoter that expresses the site-specific recombinase gene in a cell of the inner cell mass (ICM) but not the trophectoderm; and
(ii) a gene encoding a toxin, operably linked to a promoter, wherein the toxin gene is present at an expression-permissive locus; and
(iii) a nucleic acid sequence that prevents expression of the gene encoding the toxin, wherein the nucleic acid sequence is located between the promoter for the gene encoding the toxin and the coding sequence of the gene encoding the toxin and is flanked on both sides by recombinase recognition sites such that in the presence of the site-specific recombinase the nucleotide sequence is removed and the promoter for the gene encoding the toxin drives transcription of the gene encoding the toxin;
(b) gestating the embryo of step (a) in a pseudopregnant mouse.

3. The method of claim 2, wherein following gestation in the pseudopregnant mouse, a mouse pup is born, wherein the mouse pup is fully derived from the donor cell.

4. The mouse embryo of claim 1 or the method of claim 2, wherein the site-specific recombinase gene encodes a Cre recombinase, the gene whose expression prevents proliferation of the ICM cell is a gene encoding diphteria toxin A fragment (DTA), and the embryo an 8-cell stage embryo.

5. The mouse embryo of claim 1 or the method of claim 2, wherein the embryo stage is an 8-cell stage.

6. The mouse embryo of claim 1, wherein the embryo stage is selected from a pre-morula, a morula, and a blastocyst, optionally wherein the blastocyst substantially lacks a primitive endoderm.

7. The mouse embryo of claim 1 or the method of claim 2, wherein the toxin is DTA.

8. The mouse embryo of claim 1 or the method of claim 2, wherein the site-specific recombinase is a Cre recombinase or a modified Cre recombinase.

9. A breeding pair of genetically modified mice that, when mated, generate an embryo comprising a first nucleic acid construct and a second nucleic acid construct in its genome, said breeding pair comprising a first mouse comprising a first nucleic acid construct in its genome and a second mouse comprising a second nucleic acid construct in its genome,
wherein the first nucleic acid construct comprises a site-specific recombinase gene operably linked to a Nanog promoter that expresses the site-specific recombinase gene in a cell of the inner cell mass (ICM) but not the trophectoderm, and
wherein the second nucleic acid construct comprises a gene encoding a toxin and a nucleic acid sequence that prevents expression of the gene encoding the toxin, wherein the gene encoding the toxin is operably linked to a promoter and present at an expression-permissive locus, wherein the nucleic acid sequence is located between the promoter for the gene encoding the toxin and the coding sequence of the gene encoding the toxin and is flanked on both sides by recombinase recognition sites such that in the presence of the site-specific recombinase the nucleotide sequence is removed and the promoter for the gene encoding the toxin drives transcription of the gene encoding the toxin.

10. A mouse comprising the embryo of claim 1.

## Patentansprüche

1. Mäuseembryo, umfassend eine Zelle, die in ihrem Genom Folgendes hat:
(a) ein ortsspezifisches Rekombinase-Gen, das mit einem Nanog-Promoter, der das ortsspezifische Rekombinase-Gen in einer Zelle der inneren Zellmasse (ICM), jedoch nicht des Trophectoderms, exprimiert, operabel verbunden ist;
(b) ein Gen, das für ein Toxin kodiert, operabel verbunden mit einem Promoter, wobei das Toxin-Gen an einem Expression erlaubenden Locus vorliegt; und
(c) eine Nukleinsäuresequenz, die die Expression des Gens, das für das Toxin kodiert, verhindert, wobei die Nukleinsäuresequenz sich zwischen dem Promoter für das Gen, das für das Toxin kodiert, und der kodierenden Sequenz des Gens, das für das Toxin kodiert, befindet und auf beiden Seiten von Rekombinaseerkennungsstellen flankiert ist, sodass in Anwesenheit der ortsspezifischen Rekombinase die Nukleotidsequenz entfernt wird und der Promoter für das Gen, das für das Toxin kodiert, Transkription des Gens, das für das Toxin kodiert, steuert.

2. Verfahren zum Herstellen einer Maus oder eines Mäuseembryos aus einer Maus-Donorzelle und einem Wirtsembryo, umfassend:
(a) Einführen der Maus-Donorzellen in einen Mäuse-Wirtsembryo, wobei die Maus-Donorzellen aus der Gruppe bestehend aus embryonalen Stammzellen (ES), pluripotenten Stammzellen (PS) oder induzierten pluripotenten Stammzellen (iPS) ausgewählt sind und wobei der Wirtsembryo ein Prä-Morula-Stadien-Embryo ist und Folgendes umfasst:
(i) ein ortsspezifisches Rekombinase-Gen, das mit einem Nanog-Promoter, der das ortsspezifische Rekombinase-Gen in einer Zelle der inneren Zellmasse (ICM), jedoch nicht des Trophectoderms, exprimiert, operabel verbunden ist;
(ii) ein Gen, das für ein Toxin kodiert, operabel verbunden mit einem Promoter, wobei das Toxin-Gen an einem Expression erlaubenden Locus vorliegt; und
(iii) eine Nukleinsäuresequenz, die die Expression des Gens, das für das Toxin kodiert, verhindert, wobei die Nukleinsäuresequenz sich zwischen dem Promoter für das Gen, das für das Toxin kodiert, und der kodierenden Sequenz des Gens, das für das Toxin kodiert, befindet und auf beiden Seiten von Rekombinaseerkennungsstellen flankiert ist, sodass in Anwesenheit der ortsspezifischen Rekombinase die Nukleotidsequenz entfernt wird und der Promoter für das Gen, das für das Toxin kodiert, Transkription des Gens, das für das Toxin kodiert, steuert;
(b) Reifen des Embryos von Schritt (a) in einer scheinschwangeren Maus.

3. Verfahren nach Anspruch 2, wobei anschließend an das Reifen in der scheinschwangeren Maus ein Mäusejunges geboren wird, wobei das Mäusejunge vollständig von der Donorzelle abgeleitet ist.

4. Mäuseembryo nach Anspruch 1 oder Verfahren nach Anspruch 2, wobei das ortsspezifische Rekombinase-Gen für eine Cre-Rekombinase kodiert, wobei das Gen, dessen Expression die Proliferation der ICM-Zelle verhindert, ein Gen ist, das für Diphterie-Toxin-A-Fragment (DTA) kodiert, und der Embryo ein 8-Zell-Stadien-Embryo ist.

5. Mäuseembryo nach Anspruch 1 oder Verfahren nach Anspruch 2, wobei das Embryostadium in einem 8-Zell-Stadium ist.

6. Mäuseembryo nach Anspruch 1, wobei das Embryostadium aus einer Prä-Morula, einer Morula und einer Blastozyste ausgewählt ist, optional wobei es der Blastozyste im Wesentlichen an einem primitiven Endoderm fehlt.

7. Mäuseembryo nach Anspruch 1 oder Verfahren nach Anspruch 2, wobei das Toxin DTA ist.

8. Mäuseembryo nach Anspruch 1 oder Verfahren nach Anspruch 2, wobei die ortsspezifische Rekombinase eine Cre-Rekombinase oder eine modifizierte Cre-Rekombinase ist.

9. Zuchtpaar von genetisch modifizierten Mäusen, die, wenn gepaart, einen Embryo erzeugen, der ein erstes Nukleinsäurekonstrukt in seinem Genom umfasst, wobei das Zuchtpaar eine erste Maus, die ein erstes Nukleinsäurekonstrukt in ihrem Genom umfasst, und eine zweite Maus, die ein zweites Nukleinsäurekonstrukt in ihrem Genom umfasst, umfasst,
wobei das erste Nukleinsäurekonstrukt ein ortsspezifisches Rekombinase-Gen umfasst, das mit einem Nanog-Promoter, der das ortsspezifische Rekombinase-Gen in einer Zelle der inneren Zellmasse (ICM), jedoch nicht des Trophectoderms, exprimiert, operabel verbunden ist, und
wobei das zweite Nukleinsäurekonstrukt ein Gen, das für ein Toxin kodiert, und eine Nukleinsäuresequenz, die die Expression des Gens, das für das Toxin kodiert, umfasst, wobei das Gen, das für das Toxin kodiert, operabel mit einem Promoter verbunden ist und an einem Expression erlaubenden Locus vorliegt,
wobei die Nukleinsäuresequenz sich zwischen dem Promoter für das Gen, das für das Toxin kodiert, und der kodierenden Sequenz des Gens, das für das Toxin kodiert, befindet und auf beiden Seiten von Rekombinaseerkennungsstellen flankiert ist, sodass in Anwesenheit der ortsspezifischen Rekombinase die Nukleotidsequenz entfernt wird und der Promoter für das Gen, das für das Toxin kodiert, Transkription des Gens, das für das Toxin kodiert, steuert.

10. Maus, umfassend den Embryo nach Anspruch 1.

## Revendications

1. Embryon de souris, comprenant une cellule ayant, dans son génome :
(a) un gène de recombinase spécifique d'un site opérationnellement lié à un promoteur Nanog qui exprime le gène de recombinase spécifique d'un site dans une cellule de la masse cellulaire interne (MCI), mais pas du trophectoderme ;
(b) un gène codant pour une toxine, opérationnellement lié à un promoteur, le gène de toxine étant présent au niveau d'un locus permettant une expression ; et
(c) une séquence d'acides nucléiques qui empêche l'expression du gène codant pour la toxine, la séquence d'acides nucléiques étant située entre le promoteur pour le gène codant pour la toxine et la séquence de codage du gène codant pour la toxine et étant bordé des deux côtés par des sites de reconnaissance de recombinase de sorte qu'en présence de la recombinase spécifique d'un site, la séquence nucléotidique soit éliminée et que le promoteur pour le gène codant pour la toxine commande la transcription du gène codant pour la toxine.

2. Procédé de fabrication d'une souris ou d'un embryon de souris à partir d'une cellule donneuse de souris et d'un embryon hôte, comprenant :
(a) l'introduction de cellules donneuses de souris dans un embryon hôte de souris, les cellules donneuses de souris étant choisies dans le groupe constitué par des cellules souches embryonnaires (ES), des cellules souches pluripotentes (PS) ou des cellules souches pluripotentes induites (iPS), et l'embryon hôte étant un embryon de stade pré-morula et comprenant
(i) un gène de recombinase spécifique d'un site opérationnellement lié à un promoteur Nanog qui exprime le gène de recombinase spécifique d'un site dans une cellule de la masse cellulaire interne (MCI), mais pas du trophectoderme ; et
(ii) un gène codant pour une toxine, opérationnellement lié à un promoteur, le gène de toxine étant présent au niveau d'un locus permettant une expression ; et
(iii) une séquence d'acides nucléiques qui empêche l'expression du gène codant pour la toxine, la séquence d'acides nucléiques étant située entre le promoteur pour le gène codant pour la toxine et la séquence de codage du gène codant pour la toxine et étant bordé des deux côtés par des sites de reconnaissance de recombinase de sorte qu'en présence de la recombinase spécifique d'un site, la séquence nucléotidique soit éliminée et que le promoteur pour le gène codant pour la toxine commande la transcription du gène codant pour la toxine ;
(b) la gestation de l'embryon de l'étape (a) chez une souris pseudogestante.

3. Procédé de la revendication 2, dans lequel, après la gestation de la souris pseudogestante, naît un souriceau, le souriceau étant entièrement dérivé de la cellule donneuse.

4. Embryon de souris de la revendication 1 ou procédé de la revendication 2, dans lequel le gène de recombinase spécifique d'un site code pour une recombinase Cre, le gène dont l'expression empêche la prolifération de la cellule de MCI est un gène codant pour le fragment A de la toxine diphtérique (DTA), et l'embryon étant un embryon au stade octo-cellulaire.

5. Embryon de souris de la revendication 1 ou procédé de la revendication 2, dans lequel le stade de l'embryon est un stade octo-cellulaire.

6. Embryon de souris de la revendication 1, dans lequel le stade d'embryon est choisi parmi pré-morula, morula et blastocyte, le blastocyte étant éventuellement dépourvu d'endoderme primitif.

7. Embryon de souris de la revendication 1 ou procédé de la revendication 2, dans lequel la toxine est la DTA.

8. Embryon de souris de la revendication 1 ou procédé de la revendication 2, dans lequel la recombinase spécifique d'un site est une recombinase Cre ou une recombinase Cre modifiée.

9. Paire de reproduction de souris génétiquement modifiées qui, lorsqu'elles sont accouplées, engendrent un embryon comprenant un premier produit de construction d'acides nucléiques et un deuxième produit de construction d'acides nucléiques dans son génome, ladite paire de reproduction comprenant une première souris comprenant un premier produit de construction d'acides nucléiques dans son génome et une deuxième souris comprenant un deuxième produit de construction d'acides nucléiques dans son génome,
le premier produit de construction d'acides nucléiques comprenant un gène de recombinase spécifique d'un site opérationnellement lié à un promoteur Nanog qui exprime le gène de recombinase spécifique d'un site dans une cellule de la masse cellulaire interne (MCI), mais pas du trophectoderme, et
le deuxième produit de construction d'acides nucléiques comprenant un gène codant pour une toxine et une séquence d'acides nucléiques qui empêche l'expression du gène codant pour la toxine, le gène codant pour la toxine étant opérationnellement lié à un promoteur et présent au niveau d'un locus permettant une expression,
la séquence d'acides nucléiques étant située entre le promoteur pour le gène codant pour la toxine et la séquence de codage du gène codant pour la toxine et étant bordé des deux côtés par des sites de reconnaissance de recombinase de sorte qu'en présence de la recombinase spécifique d'un site, la séquence nucléotidique soit éliminée et que le promoteur pour le gène codant pour la toxine commande la transcription du gène codant pour la toxine.

10. Souris comprenant l'embryon de la revendication 1.
